# EUROPÄISCHE PATENTSCHRIFT

(11) **EP 1 286 763 B1**
(45) Veröffentlichungstag und Bekanntmachung des Hinweises auf die Patenterteilung: **08.12.2004**
(21) Anmeldenummer: 01936313.4
(22) Anmeldetag: 03.05.2001
(51) Int. Cl.: B01J 19/24, B01J 19/00, F28F 27/02, C07D 401/06, C07D 401/12, C07D 401/14, C07D 405/14

(54) **GEGENSTROM-REAKTOR MIT EINEM KONTAKTROHRBÜNDEL**
COUNTERFLOW REACTOR WITH A BUNDLE OF CONTACT TUBES
REACTEUR A CONTRE-COURANT DOTE D'UN FAISCEAU TUBULAIRE DE CONTACT

(30) Priorität: 17.05.2000 DE 10024342
(43) Veröffentlichungstag der Anmeldung: 05.03.2003
(73) Patentinhaber: BASF AKTIENGESELLSCHAFT, 67056 Ludwigshafen (DE)
(72) Erfinder: OLBERT, Gerhard, 69221 Dossenheim (DE); CORR, Franz, 67067 Ludwigshafen (DE)
(74) Vertreter: Isenbruck, Günter, Dr.
(86) Internationale Anmeldenummer: PCT/EP2001/004975
(87) Internationale Veröffentlichungsnummer: WO 2001/087476

(56) Entgegenhaltungen:
- DE-A- 19 836 792
- DE-C- 402 529
- GB-A- 667 167
- US-A- 2 345 423
- US-A- 3 760 870
- US-A- 3 871 445

## Beschreibung

Die Erfindung betrifft einen Reaktor mit einem Kontaktrohrbündel, durch dessen die Kontaktrohre umgebenden Raum ein Wärmetauschmittelkreislauf geleitet wird, sowie die Verwendung des Reaktors zur Durchführung von Oxidationsreaktionen. Ein äknlicher Reaktor wird in DE 198 36 792 beschrieben.

Die übliche Bauart gattungsgemäßer Reaktoren besteht aus einem, in der Regel zylinderförmigen Behälter, in dem ein Bündel, d. h. eine Vielzahl von Kontaktrohren, in üblicherweise vertikaler Anordnung untergebracht ist. Diese Kontaktrohre, die gegebenenfalls geträgerte Katalysatoren enthalten können, sind mit ihren Enden in Rohrböden abdichtend befestigt und münden in jeweils eine am oberen bzw. am unteren Ende mit dem Behälter verbundene Haube. Über diese Hauben wird das die Kontaktrohre durchströmende Reaktionsgemisch zu- bzw. abgeführt. Durch den die Kontaktrohre umgebenden Raum wird ein Wärmetauschmittelkreislauf geleitet, um die Wärmebilanz, insbesondere bei Reaktionen mit starker Wärmetönung, auszugleichen.

Aus wirtschaftlichen Gründen werden Reaktoren mit einer möglichst großen Zahl von Kontaktrohren eingesetzt, wobei die Zahl der untergebrachten Kontaktrohre häufig im Bereich von 10000 bis 50000 liegt.

Bezüglich des Wärmetauschmittelkreislaufs ist es bekannt, in jedem waagerechten Schnitt des Reaktors eine weitgehend homogene Temperaturverteilung des Wärmetauschmittels zu realisieren, um möglichst alle Kontaktrohre gleichmäßig am Reaktionsgeschehen zu beteiligen, (vgl. DE-C 16 01 162). Der Glättung der Temperaturverteilung dient die Wärmezuführung bzw. Wärmeabführung über jeweils an den Reaktorenden angebrachten äußeren Ringleitungen mit einer Vielzahl von Mantelöffnungen, wie sie beispielsweise in DE-C 34 09 159 beschrieben sind.

Eine weitere Verbesserung des Wärmeüberganges wird durch den Einbau von Umlenkscheiben erreicht, die abwechselnd in der Reaktormitte und am Reaktorrand einen Durchtrittsquerschnitt freilassen. Eine derartige Anordnung ist insbesondere für ringförmig angeordnete Rohrbündel mit einem freien zentralen Raum geeignet und beispielsweise aus GB-B- 310 175 bekannt.

In großen Reaktoren mit einer Zahl von Kontaktrohren im oben angegebenen Bereich von etwa 10000 bis 50000, die zusätzlich mit Umlenkscheiben ausgestattet sind, ist der Druckverlust des Wärmetauschmittels vergleichsweise sehr groß. So muß die zum Abtransport der bei Oxidationsreaktionen freiwerdenden Wärme häufig verwendete eutektische Salzschmelze von Kaliumnitrat und Natriumnitrit, die bei einer Anwendungstemperatur von bevorzugt ca. 250°C bis 400°C eine wasserähnliche Viskosität aufweist, in einen Reaktor der oben erwähnten Größe mit einer Förderhöhe von ca. 4 bis 5 m gepumpt werden, um den Druckverlust zu überwinden.

Zweckmäßigerweise wird bei derartigen großen Reaktoren das Pumpsystem zwischen der oberen und der unteren Ringleitung angeordnet, wobei das Wärmetauschmittel in den unteren Bereich des Reaktors, beispielsweise über eine Ringleitung, zugeführt wird.

Die Führung des Reaktionsgemisches erfolgt üblicherweise durch die mit Katalysator gefüllten Kontaktrohre von oben nach unten. Mit fortschreitender Reaktion in den Kontaktrohren bildet sich ein Temperaturlängsprofil aus, mit einem Temperaturmaximum (Hot-spot) im Anfangsbereich der vom Reaktionsgemisch durchströmten Rohre. Problematisch ist hierbei insbesondere, daß mit fortschreitender Betriebsdauer die Aktivität des Katalysators abfällt und in der Folge der Hot-spot-Bereich von oben nach unten durch die Reaktionsrohre wandert.

Es war daher die Aufgabe der Erfindung, ausgehend von einem einzigen äußeren Wärmetauschmittelkreislauf einen über die Reaktorhöhe und den Reaktionsfortschritt regelbaren Wärmetauschmittelfluß zu gewährleisten.

Die Erfindung geht aus von einem Reaktor mit einem Kontaktrohrbündel, durch dessen die Kontaktrohre umgebenden Raum ein Wärmetauschmittelkreislauf geleitet wird, mit Ringleitungen an beiden Reaktorenden mit Mantelöffnungen für die Zu- bzw. Abführung eines Wärmetauschmittels mittels einer oder mehrerer Pumpen, gegebenenfalls unter Überleitung des Wärmetauschmittels oder eines Teilstroms des Wärmetauschmittels über einen oder mehrere außenliegende Wärmetauscher, wobei das Wärmetauschmittel der unteren Ringleitung zugeführt und über die obere Ringleitung zur (zu den) Pumpe(n) zurückgeführt wird, sowie mit Umlenkscheiben, die abwechselnd in der Reaktormitte und am Reaktorrand einen Durchtrittsquerschnitt freilassen.

Die Lösung ist dadurch gekennzeichnet, daß die untere Ringleitung mittels einer horizontalen Trennwand in zwei Bereiche aufgeteilt ist, die miteinander über vorzugsweise gleichmäßig verteilte Öffnungen kommunizieren.

Eine weitere Lösungsvariante ist dadurch gekennzeichnet, daß mindestens eine radiale Kammer, die sich über die gesamte Reaktorhöhe erstreckt, vorgesehen ist, mit einer regelbaren Zuführöffnung für das Wärmetauschmitttel über die untere Ringleitung und einem zentralen Auslass für das Wärmetauschmittel in regelbarer Höhe in der Reaktormitte.

Die erfindungsgemäßen Reaktoren gewährleisten mit einfachen konstruktiven Maßnahmen eine erhöhte Betriebssicherheit verbunden mit einer Verlängerung der Katalysatorlebensdauer sowie einer Kapazitäts- und Selektivitätserhöhung.

Dazu wird erfindungsgemäß die untere Ringleitung, über die das Wärmetauschmittel dem Reaktor zugeführt wird, mittels einer horizontalen Trennwand in zwei Bereiche aufgeteilt, die miteinander über Öffnungen verbunden sind. Die horizontale Trennwand kann dabei grundsätzlich in beliebiger Höhe in der unteren Ringleitung angeordnet sein, bevorzugt ist jedoch eine symmetrische Aufteilung in zwei gleiche Bereiche. Ebenso können die in der horizontalen Trennwand anzuordnenden Öffnungen grundsätzlich beliebig in Anzahl und Größe sein, bevorzugt sind sie jedoch gleichmäßig verteilt angeordnet. Die Geometrie der Öffnungen ist bevorzugt kreisförmig, wobei sich die Öffnungen besonders bevorzugt nach oben konisch erweitern. Die Öffnungen weisen vorzugsweise einen regelbaren Durchtrittsquerschnitt auf, wobei zur Regelung in bekannter Weise über einen Regelantrieb steuerbare Schieber oder Ventile eingesetzt werden. Möglich sind jedoch auch Öffnungen mit festem Durchtrittsquerschnitt. Die Anzahl und Größe der Öffnungen ist vorzugsweise insgesamt so bemessen, daß ein Anteil von 0 bis 70 %, bevorzugt von 20 bis 50 % des Wärmetauschmittelflusses unmittelbar in den oberen Bereich der unteren Ringleitung einströmt.

Gemäß einer bevorzugten Ausführungsform sind im Bereich zwischen unterer Ringleitung und oberer Ringleitung über die Reaktorhöhe verteilt eine oder mehrere weitere Ringleitungen für die Zuführung jeweils regelbare Teilströme des Wärmetauschmittelstromes aus der unteren Ringleitung über regelbare Öffnungen in der horizontalen Trennwand der unteren Ringleitung sowie geeignete Zuleitungen zu den weiteren Ringleitungen vorgesehen. Besonders bevorzugt sind die weiteren Ringleitungen symmetrisch über der Reaktorhöhe verteilt. Weiter bevorzugt werden zwei oder drei weitere Ringleitungen eingesetzt. Die abzutrennenden Teilströme können grundsätzlich jeweils einen beliebigen Anteil des der unteren Ringleitung zugeführten Wärmetauschmittelstromes darstellen. Besonders bevorzugt wird jeweils ein Anteil von 0 bis 70 %, bevorzugt von 20 bis 30 % des zugeführten Wärmetauschmittelstromes abgetrennt.

Gemäß einer weiteren Ausführungsvariante ist eine radiale Kammer vorgesehen, die sich über die gesamte Reaktorhöhe erstreckt, mit einer regelbaren Zuführöffnung für das Wärmetauschmittel über die untere Ringleitung und einem zentralen Auslass für das Wärmetauschmittel in vorgebbarer Höhe in der Reaktormitte. Die technische Umsetzung dieser Ausführungsform erfolgt in besonders einfacher Weise dadurch, daß entlang eines Reaktordurchmessers in grundsätzlich beliebiger Breite, bevorzugt in einer Breite im Bereich von 100 mm bis 500 mm, insbesondere von 100 mm, ein rohrfreier Raum über die gesamte Reaktorhöhe vorgesehen ist. Die Zuführung des Wärmetauschmittels in diesen rohrfreien Raum erfolgt am Reaktorumfang, über den unteren Ringkanal, über Öffnungen mit regelbarem Durchtrittsquerschnitt. Die Regelung der Wärmetauschmittelzuführung erfolgt dabei bevorzugt mittels eines Stellantriebs, der ein Absperrorgan, insbesondere einen Schieber oder ein Ventil aufweist.

Im Bereich zwischen den Reaktionsrohren ist eine vollständige Abtrennung des Wärmetauschmittels nicht erforderlich. Bereits die Kontaktrohre selbst, die in der Regel aus Kostengründen in höchstmöglicher Dichte, in der Regel mit einem Verhältnis von Teilung, d. h. einem Abstand zwischen zwei nächstliegenden Rohren, zu Rohraußendurchmesser im Bereich von 1,26 bis etwa 1,6, eingebaut werden, bewirken aus strömungstechnischen Gründen eine weitgehende Lenkung des überwiegenden Anteils des Wärmetauschmittelstromes in die rohrfreie Mitte des Reaktors. Zusätzlich ist es möglich, parallele Seitenwände zur Abtrennung der Kammer vom mit Kontaktrohren bestückten Reaktorraum vorzusehen, es ist jedoch nicht erforderlich, diesselben flüssigkeitsdicht auszubilden.

Der zentrale Auslass des Wärmetauschmittels in der Reaktormitte kann grundsätzlich in jeder beliebigen Höhe angeordnet sein, wobei insbesondere die Anpassung an das Temperaturprofil der durchzuführenden Reaktion hierfür maßgeblich ist.

Besonders bevorzugt wird die Regelung des Wärmetauschmittelflusses über den unteren Ringkanal mittels einer horizontalen Trennwand bzw. über zusätzliche Ringkanäle über die Reaktorhöhe mit der Regelung des Wärmetauschmittelflusses über die Reaktormitte mittels einer radialen Kammer kombiniert.

Es ist auch möglich, eine weitere radiale Kammer vorzusehen, die zur ersten radialen Kammer in einem Winkel von 90° angeordnet ist, so daß sich eine Überkreuzanordnung ergibt.

In bevorzugter Weise können in einem oder allen Bereichen in der Reaktormitte, in dem die Umlenkscheiben einen Durchtrittsquerschnitt freilassen, statische Mischer angeordnet werden. Dadurch wird eine erhöhte Isothermie über den Reaktorquerschnitt erreicht, insbesondere werden Ungleichförmigkeiten der Temperaturverteilung über die beiden Reaktorhälften ausgeglichen. Bezüglich der einsetzbaren statischen Mischer gibt es grundsätzlich keine Einschränkungen; besonders bevorzugt werden statische Mischer mit Einbauten in Ring- oder Scheibenform, die insbesondere perforiert oder gewellt sein können.

Der Reaktor ist nicht eingeschränkt bezüglich der Art des Wärmetauschmittels, dieses kann gleichermaßen zur Abführung von Wärme, d.h. zur Durchführung exothermer Reaktionen, wie auch für die Zuführung von Wärme an das die Kontaktrohre durchströmende Reaktionsgemisch, d.h. zur Durchführung endothermer Reaktionen, eingesetzt werden.

Der Reaktor ist besonders geeignet zur Durchführung von Oxidationsreaktionen, insbesondere zur Herstellung von Phthalsäureanhydrid, Maleinsäureanhydrid, Glyoxal, (Meth)acrolein oder (Meth)acrylsäure.

Die Erfindung wird im folgenden anhand von Ausführungsbeispielen und einer Zeichnung näher erläutert. Es zeigen im Einzelnen:
Figur 1 einen Längsschnitt durch einen Reaktor mit regelbarer Aufteilung des Wärmetauschmittelkreislaufes über die untere Ringleitung,
Figur 1a einen Querschnitt durch den Reaktor aus Figur 1,
Figur 2 einen Längsschnitt durch einen Reaktor, der beispielhaft zwei weitere Ringleitungen mit regelbarer Zuführung von Wärmetauschmittel über die Reaktorhöhe aufweist,
Figur 3 einen Längsschnitt durch einen Reaktor mit Zuführung eines regelbaren Wärmetauschmittelstromes über eine radiale Kammer in die Reaktormitte mit Querschnitt in Figur 3a,
Figur 4 eine Ausführungsform mit zwei über Kreuz angeordneten radialen Kammern mit Querschnitt in Figur 4a und
Figur 5 eine weitere Ausführungsform mit statischen Mischern in der Reaktormitte.

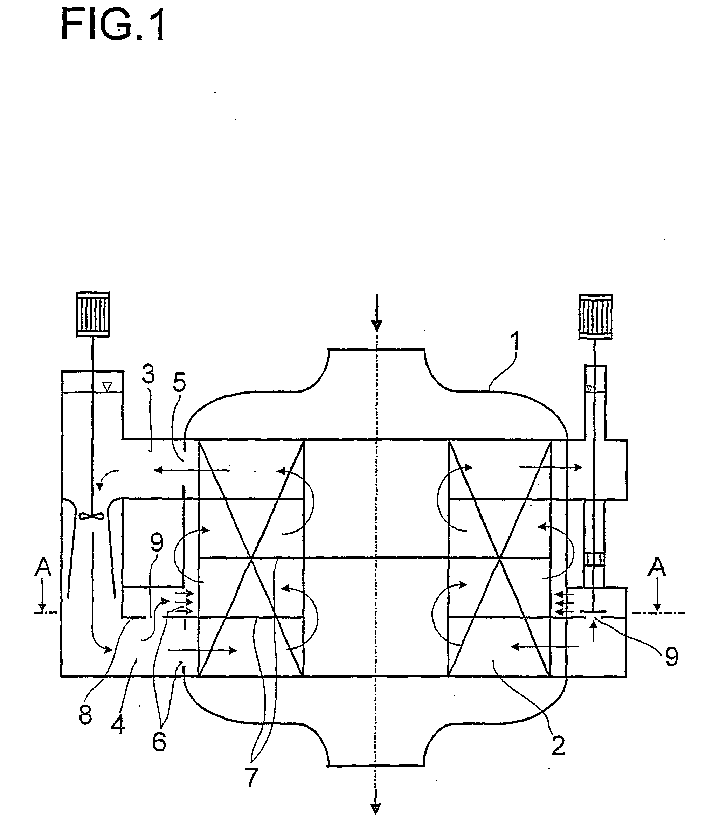

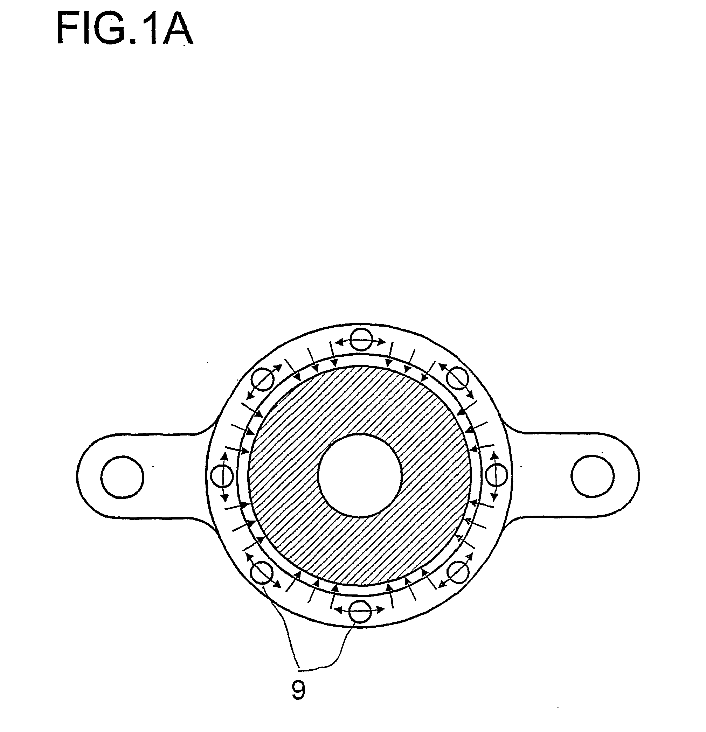

Figur 1 zeigt einen zylinderförmigen Reaktor 1 mit einem vertikalen Kontaktrohrbündel 2, das in der Zylindermitte einen Innenraum freilässt, mit unterer Ringleitung 4, der Wärmetauschmittel zugeführt wird, sowie mit oberer Ringleitung 3, über die Wärmetauschmittel abgeführt wird, wobei die Zu- bzw. Abführung des Wärmetauschmittels über Mantelöffnungen 5 und 6 erfolgt, sowie mit Umlenkscheiben 7, die einen mäanderförmigen Wärmetauschmittelkreislauf bewirken.

Insoweit ist der Aufbau des Reaktors aus dem Stand der Technik bekannt.

Erfindungsgemäß wird im Bereich der unteren Ringleitung 4 eine horizontale Trennwand 8 vorgesehen, mit Öffnungen 9 mit regelbarem Durchtrittsquerschnitt.

Wie aus dem Querschnitt in Figur 1a ersichtlich, sind die Öffnungen 9 bevorzugt symmetrisch über den Reaktorquerschnitt verteilt. Weiterhin ist aus Figur 1a die bevorzugte Geometrie der Öffnungen 9, (kreisförmig) ersichtlich.

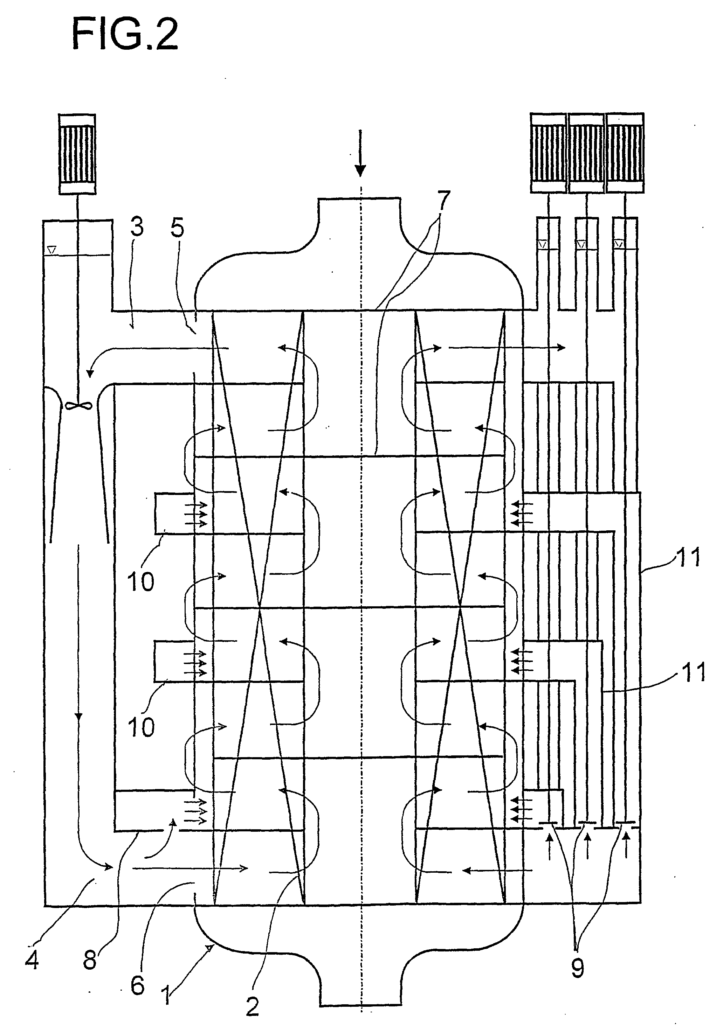

In der in Figur 2 im Längsschnitt dargestellten besonderen Ausführungsform sind zwei weitere über Reaktorhöhe bevorzugt gleichmäßig beabstandet angeordnete Ringleitungen 10 vorgesehen, die über die Zuleitungen 11 mit der unteren Ringleitung 4 über Öffnungen mit regelbarem Durchtrittsquerschnitt 9 kommunizieren.

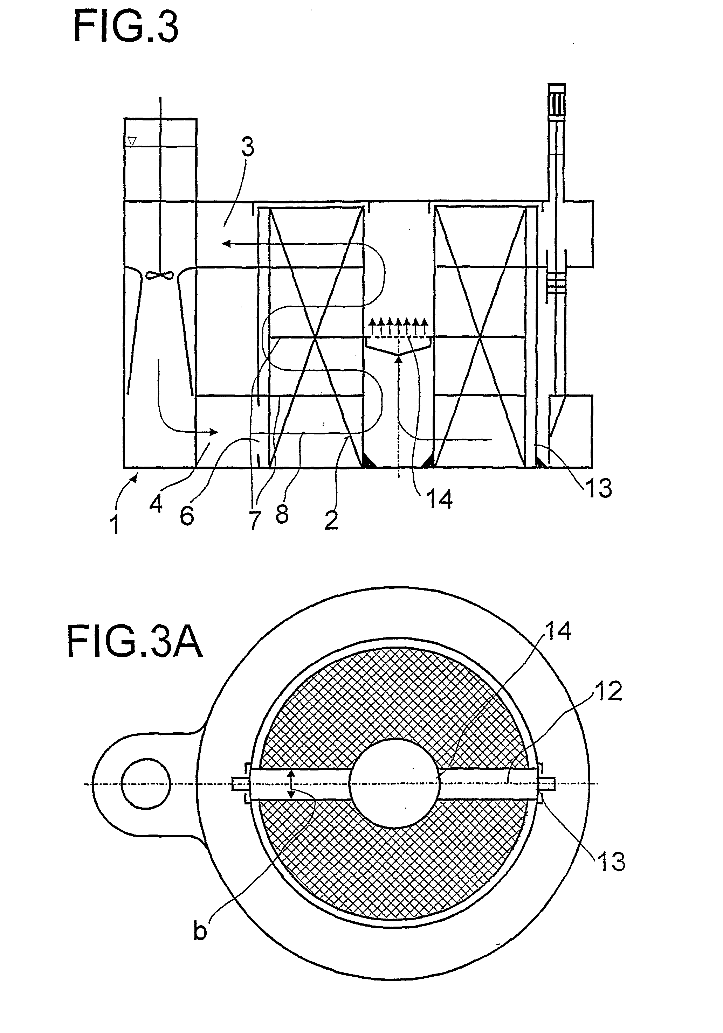

Figur 3 zeigt einen Längsschnitt durch eine Ausführungsform mit radialer Kammer 12, die sich über die gesamte Reaktorhöhe erstreckt, bevorzugt in der Breite b mit regelbarer Zuführöfnung 13 für das Wärmetauschmittel über die untere Ringleitung 4 und einem zentralen Auslass 14 für das Wärmetauschmittel in regelbarer Höhe in der Reaktormitte.

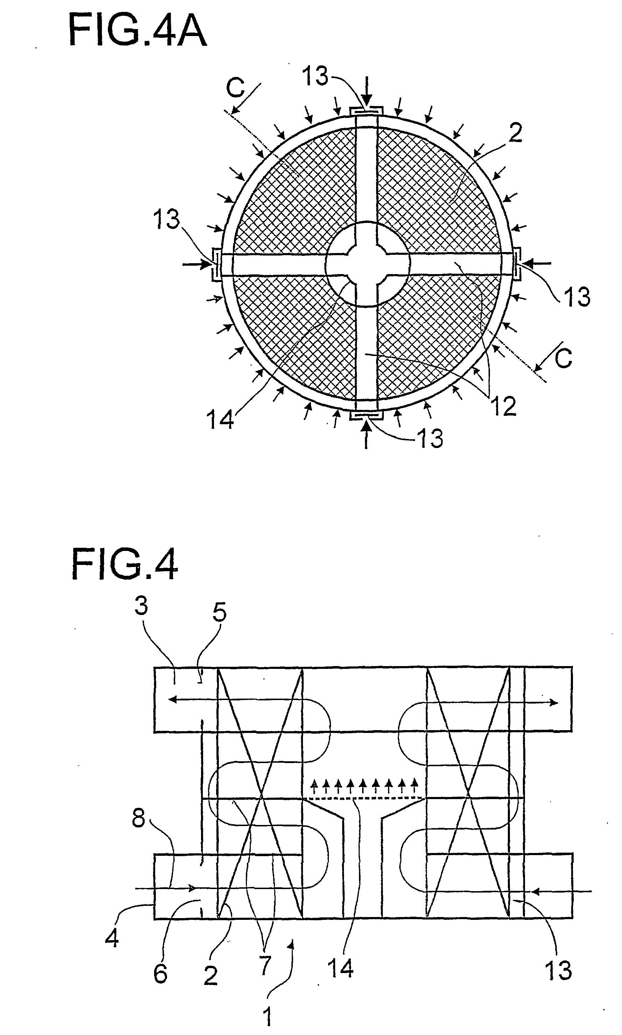

Demgegenüber zeigt Figur 4 im Längsschnitt, mit Figur 4a im Querschnitt, eine besondere Ausführungsform mit zwei über Kreuz angeordneten radialen Kammern 12.

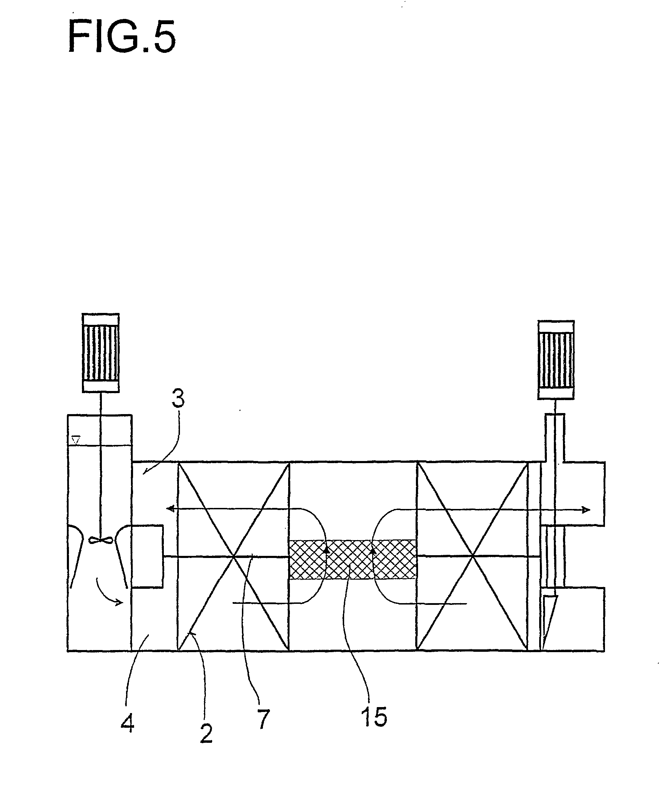

Figur 5 zeigt schematisch einen Reaktor im Längsschnitt, wobei in der Reaktormitte, in dem Bereich, in dem die Umlenkscheibe 7 einen Durchtrittsquerschnitt freilässt, ein statischer Mischer angeordnet ist.

## Patentansprüche

1. Reaktor (1) mit einem Kontaktrohrbündel (2), durch dessen die Kontaktrohre umgebenden Raum ein Wärmetauschmittelkreislauf geleitet wird, mit Ringleitungen (3, 4) an beiden Reaktorenden mit Mantelöffnungen (5, 6) für die Zu- bzw. Abführung eines Wärmetauschmittels mittels einer oder mehrerer Pumpen gegebenenfalls unter Überleitung des Wärmetauschmittels oder eines Teilstroms des Wärmetauschmittels über einen oder mehrere außenliegende Wärmetauscher, wobei das Wärmetauschmittel der unteren Ringleitung (4) zugeführt und über die obere Ringleitung (3) zur (zu den) Pumpe(n) zurückgeführt wird, sowie mit Umlenkscheiben (7), die abwechselnd in der Reaktormitte und am Reaktorrand einen Durchtrittsquerschnitt freilassen, **dadurch gekennzeichnet, daß** die untere Ringleitung (4) mittels einer horizontalen Trennwand (8) in zwei Bereiche aufgeteilt ist, die miteinander über vorzugsweise gleichmäßig verteilte Öffnungen (9) kommunizieren.

2. Reaktor (1) nach Anspruch 1, **dadurch gekennzeichnet, daß** die Öffnung (9) einen regelbaren Durchtrittsquerschnitt aufweisen.

3. Reaktor (1) nach Anspruch 1 oder 2, **dadurch gekennzeichnet, daß** im Bereich zwischen unterer Ringleitung (4) und oberer Ringleitung (3) über die Reaktorhöhe verteilt eine oder mehrere weitere Ringleitungen (10) für die Zuführung jeweils regelbarer Teilströme des Wärmetauschmittelstromes aus der unteren Ringleitung (4) über Öffnungen (9) in der horizontalen Trennwand (8) der unteren Ringleitung (4) sowie geeignete Zuleitungen (11) zu den weiteren Ringleitungen (10) vorgesehen sind.

4. Reaktor (1) mit einem Kontaktrohrbündel (2), durch dessen die Kontaktrohre umgebenden Raum ein Wärmetauschmittelkreislauf geleitet wird, mit Ringleitungen (3, 4) an beiden Reaktorenden mit Mantelöffnungen (5, 6) für die Zu- bzw. Abführung eines Wärmetauschmittels mittels einer oder mehrerer Pumpen gegebenenfalls unter Überleitung des Wärmetauschmittels oder eines Teilstroms des Wärmetauschmittels über einen oder mehrere außenliegende Wärmetauscher, wobei das Wärmetauschmittel der unteren Ringleitung (4) zugeführt und über die obere Ringleitung (3) zur (zu den) Pumpe(n) zurückgeführt wird, sowie mit Umlenkscheiben (7), die abwechselnd in der Reaktormitte und am Reaktorrand einen Durchtrittsquerschnitt freilassen, **dadurch gekennzeichnet, daß** mindestens eine radiale Kammer (12), die sich über die gesamte Reaktorhöhe erstreckt, vorgesehen ist, mit einer regelbaren Zuführöffnung (13) für das Wärmetauschmittel über die untere Ringleitung (4) und einem zentralen Auslass (14) für das Wärmetauschmittel in vorgebbarer Höhe in der Reaktormitte.

5. Reaktor (1) nach Anspruch 4, **dadurch gekennzeichnet, daß** die untere Ringleitung (4) mittels einer horizontalen Trennwand (8) in zwei Bereiche aufgeteilt ist, die miteinander über vorzugsweise gleichmäßig verteilte Öffnungen (9) mit vorzugsweise regelbarem Durchtrittsquerschnitt kommunizieren.

6. Reaktor (1) nach Anspruch 5, **dadurch gekennzeichnet, daß** im Bereich zwischen unterer Ringleitung (4) und oberer Ringleitung (3) über die Reaktorhöhe verteilt eine oder mehrere weitere Ringleitungen (10) für die Zuführung jeweils regelbarer Teilströme des Wärmetauschmittelstromes aus der unteren Ringleitung (4) über vorzugsweise regelbare Öffnungen (9) in der horizontalen Trennwand (8) der unteren Ringleitung (4) sowie geeignete Zuleitungen (11) zu den weiteren Ringleitungen (10) vorgesehen sind.

7. Reaktor nach einem der Ansprüche 4 bis 6, **gekennzeichnet durch** zwei über Kreuz angeordnete radiale Kammern (12).

8. Reaktor nach einem der Ansprüche 4 bis 7, **gekennzeichnet durch** eine Breite b der Kammer(n) im Bereich von 100 mm bis 500 mm, insbesondere von 100 mm.

9. Reaktor nach einem der Ansprüche 1 bis 8, **dadurch gekennzeichnet, daß** in einem oder allen Bereichen in der Reaktormitte, in dem die Umlenkscheiben (7) einen Durchtrittsquerschnitt freilassen, statische Mischer (15) angeordnet sind.

10. Verwendung des Reaktors nach einem der Ansprüche 1 bis 9 zur Durchführung von Oxidationsreaktionen, insbesondere zur Herstellung von Phthalsäureanhydrid, Maleinsäureanhydrid, Glyoxal, (Meth)acrolein oder (Meth)acrylsäure.

## Claims

1. A reactor (1) which has a bundle of catalyst tubes (2) and in which a heat transfer medium is circulated through the space surrounding the catalyst tubes, with ring lines (3, 4) at both ends of the reactor with openings (5, 6) through the wall for introduction or removal of a heat transfer medium by means of one or more pumps, where the heat transfer medium is introduced into the lower ring line (4) and is returned via the upper ring line (3) to the pump(s) and the heat transfer medium or a substream of the heat transfer medium may, if desired, be passed over one or more external heat exchangers, and with deflecting plates (7) which alternately leave free an open cross section in the middle of the reactor and at the edge of the reactor, wherein the lower ring line (4) is divided by means of a horizontal dividing wall (8) into two regions between which material can pass via preferably uniformly distributed openings (9).

2. A reactor (1) as claimed in claim 1, wherein the openings (9) have a regulatable open cross section.

3. A reactor (1) as claimed in claim 1 or 2, wherein one or more further ring lines (10) for the introduction in each case of regulatable substreams of the heat transfer medium stream from the lower ring line (4) via openings (9) in the horizontal dividing wall (8) of the lower ring line (4) and also suitable feed lines (11) to the further ring lines (10) are provided, distributed over the height of the reactor, in the region between the lower ring line (4) and the upper ring line (3).

4. A reactor (1) which has a bundle of catalyst tubes (2) and in which a heat transfer medium is circulated through the space surrounding the catalyst tubes, with ring lines (3, 4) at both ends of the reactor with openings (5, 6) through the wall for introduction or removal of a heat transfer medium by means of one or more pumps, where the heat transfer medium is introduced into the lower ring line (4) and is returned via the upper ring line (3) to the pump(s) and the heat transfer medium or a substream of the heat transfer medium may, if desired, be passed over one or more external heat exchangers, and with deflecting plates (7) which alternately leave free an open cross section in the middle of the reactor and at the edge of the reactor, wherein at least one radial chamber (12). which extends over the entire height of the reactor and has a regulatable feed opening (13) for the heat transfer medium from the lower ring line (4) and a central outlet (14) for the heat transfer medium at a settable height in the middle of the reactor is provided.

5. A reactor (1) as claimed in claim 4, wherein the lower ring line (4) is divided by means of a horizontal dividing wall (8) into two regions between which material can pass via preferably uniformly distributed openings (9) having a preferably regulatable open cross section.

6. A reactor (1) as claimed in claim 5, wherein one or more further ring lines (10) for the introduction in each case of regulatable substreams of the heat transfer medium stream from the lower ring line (4) via preferably regulatable openings (9) in the horizontal dividing wall (8) of the lower ring line (4) and also suitable feed lines (11) to the further ring lines (10) are provided, distributed over the height of the reactor, in the region between the lower ring line (4) and the upper ring line (3).

7. A reactor as claimed in any of claims 4 to 6, which has two radial chambers (12) arranged in the form of a cross.

8. A reactor as claimed in any of claims 4 to 7, wherein the width b of the chamber (s) is in the range from 100 mm to 500 mm, in particular 100 mm.

9. A reactor as claimed in any of claims 1 to 8, wherein static mixers (15) are located in one or all regions in the middle of the reactor where the deflecting plates (7) leave free an open cross section.

10. The use of a reactor as claimed in any of claims 1 to 9, for carrying out oxidation reactions, in particular for preparing phthalic anhydride, maleic anhydride, glyoxal, (meth)acrolein and (meth)acrylic acid.

## Revendications

1. Réacteur (1) ayant un faisceau de tubes de contact (2), à travers l'espace duquel entourant les tubes de contact est réalisé un circuit d'agent échangeur de chaleur, avec des conduites circulaires (3, 4) aux deux extrémités du réacteur avec des orifices d'enveloppe (5, 6) pour l'entrée et respectivement la sortie d'un agent échangeur de chaleur au moyen d'une ou plusieurs pompes, éventuellement avec transfert de l'agent échangeur de chaleur ou d'un courant partiel de l'agent échangeur de chaleur dans un ou plusieurs échangeurs de chaleur externes, tandis que l'agent échangeur de chaleur est introduit dans la conduite circulaire inférieure (4) et est renvoyé par l'intermédiaire de la conduite circulaire supérieure (3) dans la(les) pompe(s), ainsi qu'avec des plaques déflectrices (7), qui laissent libre une section transversale de passage alternativement dans le centre du réacteur et sur la paroi du réacteur, **caractérisé par le fait que** la conduite circulaire inférieure (4) est séparée au moyen d'une paroi de séparation horizontale (8) en deux zones qui communiquent entre elles par des orifices (9) répartis de préférence uniformément.

2. Réacteur (1) selon la revendication 1, **caractérisé par le fait que** les orifices (9) présentent une section de passage réglable.

3. Réacteur (1) selon la revendication 1 ou 2, **caractérisé par le fait que** sont prévues, réparties sur la hauteur du réacteur dans la zone entre la conduite circulaire inférieure (4) et la conduite circulaire supérieure (3) une ou plusieurs autres conduites circulaires (10) pour l'introduction à chaque fois de courants partiels du courant de l'agent échangeur de chaleur provenant de la conduite circulaire inférieure (4) par l'intermédiaire d'orifices (9) dans la paroi de séparation horizontale (8) de la conduite circulaire inférieure (4), ainsi que des conduites d'alimentation appropriées (11) aboutissant dans les autres conduites circulaires (10).

4. Réacteur (1) ayant un faisceau de tubes de contact (2), à travers l'espace duquel entourant les tubes de contact est réalisé un circuit d'agent échangeur de chaleur, avec des conduites circulaires (3, 4) aux deux extrémités du réacteur avec des orifices d'enveloppe (5, 6) pour l'entrée et respectivement la sortie d'un agent échangeur de chaleur au moyen d'une ou plusieurs pompes, éventuellement avec transfert de l'agent échangeur de chaleur ou d'un courant partiel de l'agent échangeur de chaleur dans un ou plusieurs échangeurs de chaleur externes, tandis que l'agent échangeur de chaleur est introduit dans la conduite circulaire inférieure (4) et est renvoyé par l'intermédiaire de la conduite circulaire supérieure (3) dans la(les) pompe(s), ainsi qu'avec des plaques déflectrices (7), qui laissent libre une section de passage alternativement dans le centre du réacteur et sur la paroi du réacteur, **caractérisé par le fait qu'**il est prévu au moins une chambre radiale (12), qui s'étend sur la totalité de la hauteur du réacteur, ayant un orifice d'alimentation (13) réglable pour l'agent échangeur de chaleur par l'intermédiaire de la conduite circulaire inférieure (4) et une sortie centrale (14) pour l'agent échangeur de chaleur à une hauteur prédéterminable dans le centre du réacteur.

5. Réacteur (1) selon la revendication 4, **caractérisé par le fait que** la conduite circulaire inférieure (4) est divisée au moyen d'une paroi de séparation horizontale (8) en deux zones, qui communiquent entre elles par l'intermédiaire d'orifices (9) avantageusement régulièrement répartis, ayant une section transversale de passage avantageusement réglable.

6. Réacteur (1) selon la revendication 5, **caractérisé par le fait que** sont prévues, réparties dans la zone entre la conduite circulaire inférieure (4) et la conduite circulaire supérieure (3) sur la hauteur du réacteur, une ou plusieurs conduites circulaires (10) pour l'alimentation à chaque fois en courants partiels réglables du courant d'agent échangeur de chaleur provenant de la conduite circulaire inférieure (4) par l'intermédiaire d'orifices avantageusement réglables (9) dans la paroi de séparation horizontale (8) de la conduite circulaire inférieure (4), ainsi que des conduites d'alimentation (11) appropriées aboutissant aux autres conduites circulaires (10).

7. Réacteur selon l'une des revendications 4 à 6, **caractérisé par** deux chambres radiales (12) disposées en croix.

8. Réacteur selon l'une des revendications 4 à 7, **caractérisé par** une largeur b de la(des) chambre(s) de 100 mm à 500 mm, en particulier de 100 mm.

9. Réacteur selon l'une des revendications 1 à 8, **caractérisé par le fait que** dans l'une ou la totalité des zones dans le centre du réacteur dans lequel les plaques déflectrices (7) libèrent une section transversale de passage, sont placés des mélangeurs statiques (15).

10. Utilisation du réacteur selon l'une des revendications 1 à 9 pour l'exécution de réactions d'oxydation, en particulier pour la préparation d'anhydride d'acide phtalique, d'anhydride d'acide maléique, de glyoxal, de (méth)acroléine ou d'acide (méth)acrylique.
